# DEMANDE DE BREVET EUROPEEN

(11) **EP 1 892 638 A1**
(43) Date de publication de la demande: **27.02.2008**
(21) Numéro de dépôt: 07113342.5
(22) Date de dépôt: 27.07.2007
(51) Int. Cl.: G06F 19/00

(54) **Procédé de surveillance épidémiologique mis en oeuvre à l'aide d'une communauté de services réseaux**

(30) Priorité: 25.08.2006 FR 0607528
(71) Demandeur: Etat Français représenté par le Délégué Général pour L'Armement, 94114 Arcueil Cedex (FR); Université de la Méditerranée - Aix-Marseille II, 13284 Marseille Cedex 07 (FR)
(72) Inventeur: Boutin, Jean-Paul, 13190 Allauch (FR); Chaudet, Hervé, 13700 Marignane (FR); Meynard, Jean-Baptiste, 97354 Remire Montjoly (GF); Texier, Gaëtan, 13112 La Destrousse (FR)
(74) Mandataire: Domange, Maxime

(57) **Abrégé**

L'invention concerne un procédé de surveillance épidémiologique mis en oeuvre à l'aide d'une communauté de services réseau déployée au sein de plusieurs serveurs [1,2,3] aptes à communiquer entre eux et dont au moins un d'entre eux, dit serveur de données [1], met en oeuvre un service réseau de stockage [10] de données épidémiologiques en provenance d'au moins un système de déclaration [5',6] de données épidémiologiques, ladite communauté pouvant être utilisée par plusieurs clients réseau [5,5'] dont au moins un client dit de surveillance [5].

## Description

### Arrière-plan de l'invention

La présente invention se rapporte au domaine général de la surveillance épidémiologique.

A l'heure actuelle, les systèmes de surveillance épidémiologique pour détecter des épidémies et réaliser des alertes précoces sont nombreux. Ils visent en particulier à déclencher une alerte lors d'une agression biologique, que celle-ci soit naturelle ou non.

En général, ces systèmes de surveillance collectent l'activité de structures médicales sous forme de données d'activité agrégées ou sous forme de déclaration électronique effectuée lors d'une consultation médicale.

En particulier, le système américain ESSENCE surveille les signes présentés par des patients se présentant dans un ensemble de 417 structures de soins militaires réparties sur l'ensemble du globe. Ces structures de soins expédient les données récoltées selon un rythme journalier.

Dans les systèmes connus, les données sont ainsi diffusées périodiquement des structures de soins vers un serveur centralisateur qui concentre les informations et qui effectue le traitement des données. Par conséquent, le traitement de données est centralisé et effectué par un ensemble logiciel monolithique.

L'envoi de données et la qualité des données envoyées sont assujettis au comportement de l'équipe en charge de réaliser cet envoi et des moyens disponibles dans les différentes structures médicales pour organiser les données et leur contenu. La seule modularité autorisée par ces systèmes est l'équipement d'une structure médicale, accompagnée d'une formation d'un personnel déclarant qui doit consentir à divulguer ses données, pour la rendre susceptible d'envoyer des données épidémiologiques vers un serveur centralisateur.

De manière générale, à cause de la diversité des structures médicales et des personnels déclarants, les données envoyées ne sont pas présentées selon un format particulier et commun à toutes les structures médicales. Un pré traitement des données reçues s'avère donc souvent nécessaire au sein du serveur centralisateur.

Les systèmes connus de l'art antérieur permettent donc un traitement de données envoyées par des structures médicales réparties sur un territoire mais ne tiennent pas compte et ne peuvent pas tenir compte d'un certain nombre de spécificités observées quant à la surveillance de la santé dans le domaine militaire.

Parmi ces spécificités, on compte la mobilité géographique des patients et des structures médicales sources de données, le besoin de recueillir et d'analyser localement les données de santé sur le terrain et le besoin d'avoir une surveillance en temps réel pour, le cas échéant, déclencher une action locale adaptée et rapide et donc avoir une réactivité satisfaisante quant au phénomène observé.

On entend ici par les termes « temps réel », une durée courte correspondant à une période de rafraichissement des données permettant le suivi d'une épidémie sans retard préjudiciable. Une telle période peut être de quelques minutes à quelques heures.

Il existe ainsi un besoin non satisfait quant à la détection rapide des anomalies relatives à la santé des militaires déployés sur un théâtre d'opérations.

Ce besoin est d'autant plus crucial que l'on sait probable l'occurrence d'attaques biologiques par des armes de destruction massive sur de telles populations.

Globalement, l'invention concerne donc, entre autres, la lutte contre le bioterrorisme et les armes biologiques ciblant aussi bien les populations militaires que civiles et engendrant des problèmes de santé qu'il est crucial de détecter et de localiser en temps réel.

Or, aucun des systèmes connus ne permet de réaliser un traitement en temps réel des données. Aucun des systèmes connus ne prend en compte le besoin de connaître les résultats du traitement de données en local, par exemple sur un théâtre d'opérations. Enfin, aucun système connu ne permet de modifier la nature des traitements informatiques, l'implantation géographique des machines, la capacité de traitement sans arrêter le dispositif de surveillance, et ne permet la redondance des ressources informatiques qui sont une nécessité en milieu militaire.

### Objet et résumé de l'invention

La présente invention a pour but principal de palier à de tels inconvénients en proposant un procédé de surveillance épidémiologique mis en oeuvre à l'aide d'une communauté de services réseau déployée au sein de plusieurs serveurs aptes à communiquer entre eux et dont au moins un d'entre eux, dit serveur de données, met en oeuvre un service réseau de stockage de données épidémiologiques en provenance d'au moins un système de déclaration de données épidémiologiques, ladite communauté pouvant être utilisée par plusieurs clients réseau dont au moins un client dit de surveillance, comprenant au moins les étapes de :
- pour le client de surveillance, activer un service réseau de surveillance épidémiologique,
- pour le service réseau de surveillance épidémiologique, activer au moins un service réseau apte à envoyer une requête au serveur de données pour la sélection et l'envoi de données épidémiologiques,
- pour le serveur de données, recevoir la requête, la transférer au service réseau de stockage qui réalise la sélection de données épidémiologiques, et retourner les données épidémiologiques sélectionnées,
- pour le service réseau de surveillance, activer au moins un service réseau de traitement apte à traiter les données épidémiologiques reçues en retour par le service réseau apte à envoyer une requête au serveur de données, et retourner le résultat du traitement au client de surveillance,
- pour le client de surveillance, afficher, le cas échéant, un résultat du traitement sur une interface homme-machine.

Ainsi, avec l'invention, le serveur de données met à disposition les données issues de la surveillance épidémiologique pour la communauté de service réseau susceptible d'être utilisée par le client réseau. Les données sont demandées au besoin par ces services.

Cette communauté peut être utilisée à l'aide de plusieurs clients réseau adaptés aux différents métiers des utilisateurs.

Avec un tel procédé, il est possible de répartir les diverses fonctions de traitement de données sur plusieurs serveurs. Cela permet d'ajouter, de supprimer ou de modifier certaines fonctions du traitement de données de manière simple et aisée.

L'invention permet à tout moment d'ajouter un site de surveillance épidémiologique (par exemple un nouveau théâtre d'opération) en connectant simplement sur le réseau commun un serveur de données pour le nouveau site de recueil, du moment que celui-ci dispose des services réseau capables de dialoguer automatiquement avec la communauté de services réseau. Les moyens de télécommunication constituant le réseau peuvent être divers moyens matériels dont les moyens filaires, par ondes radio (par exemple des moyens de téléphonie mobile) ou encore satellitaire.

Dès qu'un tel serveur possède au moins un service réseau de requête et au moins un service réseau d'analyse ou un accès à un service réseau de traitement mis en oeuvre sur un autre serveur, il peut commander des analyses de données en local ou distant sur un autre serveur du réseau.

L'invention offre aussi, par l'usage d'un service dédié à envoyer des requêtes à un service réseau de stockage, l'avantage de permettre une mise à jour automatique et périodique des données épidémiologiques utilisées pour les analyses indépendamment de l'envoi de données par un personnel déclarant.

Selon une caractéristique particulière de l'invention, le client de surveillance active périodiquement le service réseau de surveillance.

Dans un mode de réalisation de l'invention, le service réseau de traitement est apte à envoyer lui-même une requête au serveur de données.

Avec une telle caractéristique, le service réseau de requête est alors également le service réseau de traitement.

Dans un mode de réalisation, le service réseau de surveillance est apte à envoyer lui-même une requête au serveur de données.

Avec une telle caractéristique, dès que le service réseau de surveillance est activé, des données sont requises auprès du serveur de données. Ces données sont alors ensuite envoyées vers le service réseau de traitement lors de son activation.

Selon une caractéristique avantageuse, un serveur de données est mis en oeuvre pour chaque site surveillé.

Cette caractéristique permet de placer le stockage des données le plus en amont possible vers la source de données en répartissant l'information le plus possible et en permettant des mises à jour locales les plus rapides et les plus aisées. Comme les données stockées dans les serveurs locaux sont directement celles utilisées dans les traitements de données, on garantit ainsi une actualisation des données optimale.

Dans une mise en oeuvre avantageuse, les données épidémiologiques sont des n-uplets comprenant 3 axes et des attributs.

Dans un mode de réalisation, le procédé comprend une étape préalable de saisie des données épidémiologiques en utilisant un logiciel de travail collaboratif et partagé.

L'invention concerne aussi une communauté de services réseau déployée au sein de plusieurs serveurs dont au moins un d'entre eux, dit serveur de données, met en oeuvre un service réseau de stockage de données épidémiologiques en provenance d'au moins un système de déclaration de données épidémiologiques, ladite communauté pouvant être utilisée par plusieurs clients réseau et étant destinée à mettre en oeuvre un procédé selon l'invention.

L'invention concerne encore un dispositif serveur réseau dit de traitement de l'information apte à communiquer avec d'autres serveur, l'ensemble de ces serveurs mettant en oeuvre une communauté de services réseau pouvant être utilisée par plusieurs clients réseau, au moins un des serveurs, dit serveur de données, mettant en oeuvre un service réseau de stockage de données épidémiologiques en provenance d'au moins un système de déclaration de données épidémiologiques, ledit serveur de traitement de l'information comprenant un service réseau de surveillance apte à activer un service réseau apte à envoyer une requête au serveur de données pour la sélection et l'envoi de données épidémiologiques et apte à activer au moins un service réseau de traitement des données épidémiologiques sélectionnées pour traiter les données épidémiologiques.

Une telle structure minimum de serveur de traitement de l'information permet d'ajouter des traitements de données particuliers sous forme de services réseau et ce, aisément, éventuellement localement, sans avoir à reconfigurer l'ensemble du procédé.

L'invention concerne encore un serveur réseau dit de données apte à communiquer avec d'autres serveurs, l'ensemble de ces serveurs mettant en oeuvre une communauté de services réseau pouvant être utilisée par plusieurs clients réseau, le dit serveur de données mettant en oeuvre un service réseau de stockage de données épidémiologiques en provenance d'au moins un système de déclaration de données épidémiologiques et des moyens de traitement de requête en provenance d'un serveur de traitement de l'information comprenant un service réseau de surveillance apte à activer un service réseau apte à envoyer une requête au serveur de données pour la sélection et l'envoi de données épidémiologiques.

Une telle structure minimum pour un serveur réseau de données permet d'ajouter aisément au sein du système de surveillance des serveurs divers tant qu'ils incluent une base de données. En particulier, le format des données peut être divers et géré par les moyens de traitement de requête qui standardisent alors les données avant envoi.

L'invention concerne aussi un dispositif client réseau apte à communiquer avec au moins un serveur d'un ensemble de serveurs mettant en oeuvre une communauté de services réseau pouvant être utilisée par plusieurs clients réseau, le client réseau comprenant des moyens pour activer un service réseau de surveillance épidémiologique apte à activer au moins un service réseau apte à envoyer une requête à un serveur de données, le dit serveur de données mettant en oeuvre un service réseau de stockage de données épidémiologiques en provenance d'au moins un système de déclaration de données épidémiologiques, pour la sélection et l'envoi de données épidémiologiques, et apte à activer au moins un service réseau de traitement apte à traiter les données épidémiologiques reçues en retour par le service réseau apte à envoyer une requête au serveur de données, et retourner le résultat du traitement au client de surveillance et des moyens d'affichage pour afficher, le cas échéant, un résultat du traitement des données épidémiologiques.

Selon une mise en oeuvre préférée, les différentes étapes du procédé sont déterminées par des instructions de programmes d'ordinateurs.

En conséquence, l'invention vise aussi un programme d'ordinateur sur un support d'informations, ce programme étant susceptible d'être mis en oeuvre dans un dispositif serveur réseau, ce programme comportant des instructions adaptées à la mise en oeuvre des étapes suivantes :
- pour le client de surveillance, activer un service réseau de surveillance épidémiologique,
- pour le service réseau de surveillance épidémiologique, activer au moins un service réseau apte à envoyer une requête au serveur de données pour la sélection et l'envoi de données épidémiologiques,
- pour le serveur de données, recevoir la requête, la transférer au service réseau de stockage qui réalise la sélection de données épidémiologiques, et retourner les données épidémiologiques sélectionnées,
- pour le service réseau de surveillance, activer au moins un service réseau de traitement apte à traiter les données épidémiologiques reçues en retour par le service réseau apte à envoyer une requête au serveur de données, et retourner le résultat du traitement au client de surveillance,
- pour le client de surveillance, afficher, le cas échéant, un résultat du traitement sur une interface homme-machine.

Ce programme peut utiliser n'importe quel langage de programmation, et être sous la forme de code source, code objet, ou de code intermédiaire entre code source et code objet, tel que dans une forme partiellement compilée, ou dans n'importe quelle autre forme souhaitable.

L'invention vise aussi un support d'informations lisible par un dispositif serveur réseau, et comportant des instructions d'un programme d'ordinateur tel que mentionné ci-dessus.

Le support d'informations peut être n'importe quelle entité ou dispositif capable de stocker le programme. Par exemple, le support peut comporter un moyen de stockage, tel qu'une ROM, par exemple un cédérom ou une ROM de circuit microélectronique, ou encore un moyen d'enregistrement magnétique, par exemple une disquette (floppy disc) ou un disque dur.

D'autre part, le support d'informations peut être un support transmissible tel qu'un signal électrique ou optique, qui peut être acheminé via un câble électrique ou optique, par radio ou par d'autres moyens. Le programme selon l'invention peut être en particulier téléchargé sur un réseau de type Internet.

Alternativement, le support d'informations peut être un circuit intégré dans lequel le programme est incorporé, le circuit étant adapté pour exécuter ou pour être utilisé dans l'exécution du procédé en question.

### Brève description des dessins

D'autres caractéristiques et avantages de la présente invention ressortiront de la description faite ci-dessous, en référence aux dessins annexés qui en illustrent un exemple de réalisation dépourvu de tout caractère limitatif. Sur les figures :
- la figure 1 est une représentation schématique d'une communauté de serveurs selon l'invention,
- la figure 2 illustre le fonctionnement d'un client,
- la figure 3 est une représentation schématique d'une interface graphique utilisée sur des moyens d'affichage d'un client réseau de surveillance,
- la figure 4 représente trois niveaux d'alerte pour un affichage sur un client réseau de surveillance,
- la figure 5 donne un exemple d'architecture de déploiement.

### Description détaillée d'un mode de réalisation

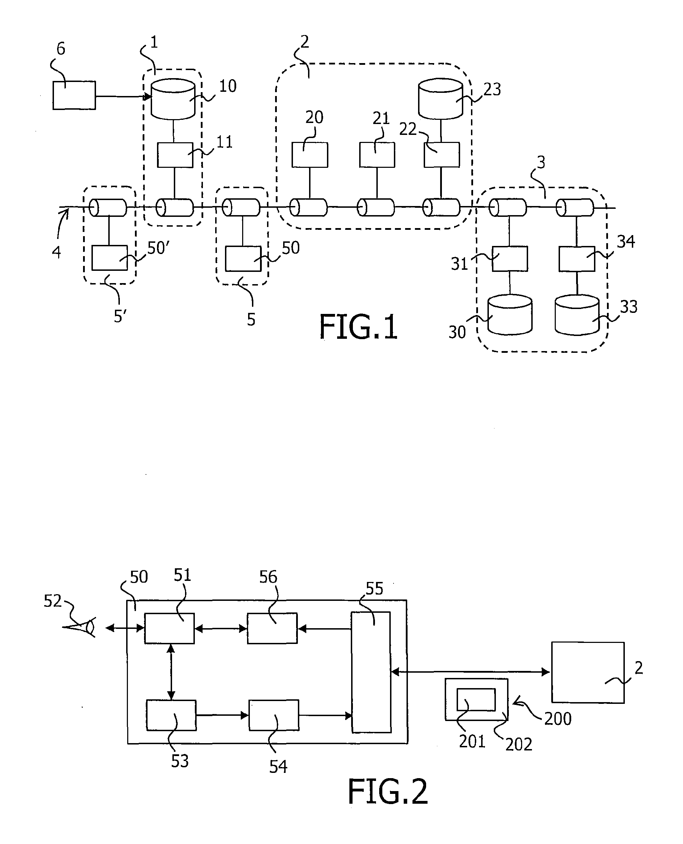

La figure 1 représente schématiquement une communauté de familles de serveurs 1, 2, 3 utiles pour réaliser un procédé selon l'invention. Ces derniers sont reliés à l'aide d'un réseau 4 auquel est également connecté au moins un client réseau de surveillance 5 réalisant une interface homme-machine.

Le réseau 4 assure la communication entre les serveurs et est, par exemple, constitué d'un réseau Internet sécurisé et/ou des réseaux intranets protégés. Les communications Internet sont avantageusement protégées, par exemple par un tunneling SSL pour Secure Sockets Layer en anglais de 128 bits minimum avec authentification X509. Le réseau 4 utilise diverses ressources matérielles de télécommunication.

La famille de serveurs 1 regroupe les serveurs de données. Au sein du serveur de données 1 représenté sur la figure 1, est mis en oeuvre un service réseau de stockage de données épidémiologiques comprenant une mémoire 10 et des moyens pour le traitement des requêtes 11. Le stockage des données épidémiologiques se fait dès leur déclaration sur un système de déclaration 6 associé au serveur de données 1 ou encore à partir d'un client réseau 5' de déclaration.

La famille de serveurs 2 rassemble les serveurs dits de traitement de l'information. Au sein du serveur de surveillance 2 sont mis en oeuvre plusieurs services réseaux permettant le traitement des données épidémiologiques stockées dans le serveur de données 1, leur organisation et leur communication à des clients d'interface.

La famille de serveurs 3 regroupe les serveurs dits d'intégration de l'ensemble de la communauté de services. Le serveur de gestion 3 représenté sur la figure 1, intègre un ensemble de moyens qui permettent l'interopérabilité des services réseau.

Préalablement à la mise en oeuvre du procédé selon l'invention, il est nécessaire que soit effectuée localement, dans les structures de santé, une déclaration de données épidémiologiques.

Il existe diverses manières de mettre en oeuvre des moyens permettant une telle déclaration. La figure 1 donne deux exemples de telles mises en oeuvre. Dans la première, le serveur de données 1 présente une interface directe avec un système de déclaration 6 permettant la déclaration de cas par un utilisateur. Les cas sont directement stockés vers la mémoire 10 dès validation. Le système de déclaration 6 peut, par exemple être une interface graphique mise en oeuvre au sein du même dispositif matériel que le serveur de données lui-même.

Dans la seconde, les moyens de déclaration sont mis en oeuvre au sein d'un client réseau 5' connecté au réseau 4. Ce client 5' comprend alors un ensemble de moyens 50 comprenant au moins une interface graphique permettant à un utilisateur de ce client 5' de saisir des données épidémiologiques sous la forme d'une fiche de cas. L'interface graphique permet au personnel d'éditer une fiche de cas, de la valider et de la transmettre après avoir précisé sa description. Ces données, une fois saisies, sont transmises directement vers le serveur de données 1 via le réseau 4.

On remarque que les données peuvent également être capturées de manière automatique, par exemple depuis un dispositif biométrique ou un autre logiciel, et indépendamment d'une quelconque action d'un utilisateur, L'envoi des données vers le serveur de données 1 via le serveur est alors aussi automatique.

La seconde mise en oeuvre au sein d'un client réseau 5' présente l'avantage d'utiliser des ressources mises en place lors du déploiement du réseau selon l'invention sans nécessiter d'éléments additionnels, hormis logiciels, pour la gestion du stockage direct dans la mémoire 10.

On note aussi ici qu'un même dispositif matériel peut comprendre un client de déclaration et un client de surveillance.

Les données épidémiologiques sont avantageusement standardisées au sein des fiches de cas. Pour cela, il est avantageux que la saisie des données épidémiologiques soit réalisée en utilisant un logiciel de travail collaboratif et partagé. Un tel logiciel, par exemple mis en oeuvre au sein du client de déclaration 5', offre la possibilité de remplir des champs prédéfinis pour caractériser les données saisies.

Ainsi, avantageusement, chaque serveur de données connecté au réseau met à disposition, de façon univoque, les données épidémiologiques sous la forme d'évènements épidémiologiques EE auxquels sont rattachés des attributs décrivant les caractéristiques des cas. Un évènement épidémiologique EE est avantageusement présenté sous la forme d'un n-uplet comprenant un identificateur unique de l'évènement rapporté (par exemple un numéro de fiche), un identifiant du groupe d'appartenance du cas (par exemple une unité ou une mission), un repère temporel et un indicateur de géo référencement, qui peut être direct sous la forme d'une latitude et d'une longitude ou indirect par une fonction nommée indiquant le géo référencement, par exemple le symbole d'un lieu ou d'une zone.

Dans l'application visée, le repère temporel est constitué avantageusement de plusieurs dates qui sont la date d'apparition des signes chez le patient, la date de consultation à laquelle le patient a rapporté les signes au personnel déclarant, la date de notification par remplissage de la fiche de déclaration au sein du logiciel de travail collaboratif et la date de transmission de la fiche par voie électronique vers le serveur de données. Chaque date est si possible un horodatage.

Outre les données du n-uplets, chaque évènement épidémiologique EE rapporte en outre des attributs que sont les caractéristiques cliniques, biologiques ou autres du cas. Ces attributs peuvent aussi inclure des résultats de dispositifs de diagnostic rapide si ces derniers sont disponibles.

Avantageusement, le contenu des déclarations correspond au minimum aux éléments précisés dans l'accord de normalisation Stanag OTAN 2277.

Les informations de déclaration relatives à un évènement épidémiologique EE sont sauvegardées sur une base de données propriétaire du logiciel de travail collaboratif du client réseau 5' puis transférées à courte échéance vers le serveur de données 1. Les données épidémiologiques sont alors stockées dans la mémoire 10 selon un schéma de base de données relationnelle particulier au serveur de données 1. Dans tous les cas, quel que soit le schéma de la base de données du serveur 1 stockée dans la mémoire 10, les informations épidémiologiques sont rendues accessibles sous la forme d'une vue correspondant à la définition d'un évènement épidémiologique EE.

Le transfert à courte échéance des données épidémiologiques définissant les évènements épidémiologiques EE à partir du système de déclaration 6 ou du client de déclaration 5' peut être instantané à la validation d'une fiche ou être réalisé automatiquement de manière périodique, par exemple toutes les deux minutes. Au sein de la mémoire 10, l'architecture de la base de données relationnelle distingue avantageusement quatre types d'entités dans le schéma relationnel. Il s'agit de la population exposée donnant l'identification des individus et la composition des unités, la description clinique de l'évènement épidémiologique EE avec son horodatage complet, la description des missions comprenant la composition et l'itinéraire des missions et la description du lieu pré-géo-référencé.

Par conséquent, une fois les évènements épidémiologiques EE stockés en temps réel dans le serveur de données 1, ils sont rendus disponibles sous la forme d'une base de données relationnelle pour des services réseau mis en oeuvre dans les serveurs connectés au réseau 4, en particulier les serveurs de traitement de l'information 2.

Afin d'accéder aux données, le serveur de traitement de l'information 2 représenté sur la figure 2 met en oeuvre un service réseau de requête pour demander l'envoi de données épidémiologiques sous forme d'évènements EE ou de champs d'évènements EE.

Dans un contexte plus général, plusieurs serveurs de surveillance et plusieurs serveurs de données sont connectés au réseau 4 et les services réseau de requête mis en oeuvre au sein des serveurs de traitement de l'information 2 interrogent successivement tous les serveurs de données 1.

Les serveurs de données interrogés et leur fréquence de requête sont avantageusement fonction des traitements demandés par l'utilisateur via le client de surveillance 5 présenté dans la suite.

Les serveurs de traitement de l'information 2 réalisent ainsi une récolte de données épidémiologiques en chainant les requêtes des différents services réseau de stockage de données épidémiologiques au sein des serveurs de données 1 et cela, avec une périodicité courte et paramétrable, par exemple cinq minutes.

Une choréographie particulière des services réseau définie au sein d'un serveur de surveillance réalisant un service réseau de surveillance 20 permet la programmation de ces requêtes et des réponses associées. En pratique, le serveur de surveillance peut être distinct de, inclus dans ou confondu avec le serveur de traitement de l'information 2. Comme illustré sur la figure 1, le serveur de surveillance est avantageusement mis en oeuvre au sein du serveur de traitement de l'information 2. Ainsi, dans la mesure où, selon l'invention, il n'y a aucune commande spécifique à réaliser par le personnel déclarant si ce n'est la déclaration elle-même dans une fiche de cas standardisée, le procédé selon l'invention permet de traiter les données en temps réel et de manière indépendante du comportement des personnels déclarants.

Toute requête à un service réseau comporte un identifiant sécurisé du demandeur. Cet identifiant est associé à une gestion des droits d'accès au service qui permet de déterminer quel traitement, avec quel type de résultat, est autorisé pour le demandeur. Cela permet entre autres de gérer les autorisations d'accès aux données en fonction d'un classement de confidentialité. Cette gestion de la confidentialité est prise en charge au niveau de la choréographie afin d'éviter les échecs de traitement.

Ce service de surveillance 20 définit ainsi avantageusement les enchaînements de requêtes de services réseau de traitements statistiques 21 ou géographiques 22 correspondant respectivement aux traitements et visualisations d'informations qui sont offerts aux clients d'interface 5.

Les traitements statistiques ou géographiques peuvent soit effectuer eux-mêmes les requêtes aux serveurs de données en fonction de leurs besoins, soit demander que le service de surveillance 20 effectue lui-même les requêtes pour fournir les données à traiter.

Concernant précisément la programmation des requêtes et des réponses, tous les services réseau communiquent selon les standards habituellement utilisé pour la structure des messages échangés entre services réseau. Par exemple, la façon d'interroger chaque service est décrite suivant le standard WSDL (Web Service Description Language), et les messages échangés entre les services sont structurés suivant le protocole SOAP (Simple Object Access Protocol).

Avantageusement, toutes les entités, ou concepts, utilisées pour définir un service en WSDL ou pour composer un message échangé entre services sont définies au niveau du serveur d'ontologie 33, qui donne ainsi accès à la définition de cette entité et permet la standardisation et l'interopérabilité des services.

Le standard WSDL définit quatre types d'opérations qui vont être utilisées au sein de la communauté de service réseau selon l'invention et dans les messages XML échangés. Il s'agit des opérations en sens unique, lorsque le service ou client réseau reçoit des messages mais n'en n'envoie pas, en réponse à requête, lorsque le service ou client réseau reçoit des messages et renvoie des messages, en sollicitation de réponse, lorsque le service ou client réseau envoie des messages puis en reçoit, en notification, lorsque le service ou client réseau envoie des messages mais n'en reçoit pas.

Les échanges entre les services et clients réseau sont donc réalisés en fonction des définitions des services réseau en norme WSDL qui sont proposées par chaque serveur. L'ensemble de la communication de données épidémiologiques entre les serveurs se fait donc de manière automatique selon des caractéristiques préalablement définies dans chacun des services réseau gérant l'appel et l'envoi de données.

En l'occurrence, ici, le service réseau de surveillance 20 fonctionne en sollicitation de réponse vis-à-vis du serveur de données 1 qui, lui, fonctionne alors en réponse à requête.

La sollicitation de réponse par le service réseau de requête comporte un identifiant du service réseau interrogé, par exemple le service réseau de stockage du serveur de données 1, un ensemble de données sur lequel porte la requête, notamment, la définition par ses attributs des évènements à sélectionner, la définition de la population sélectionnée, la définition de la période temporelle sélectionnée, la définition de la région spatiale sélectionnée, une éventuelle requête complémentaire sur les attributs, les champs à retourner en réponse et une instruction de groupage des résultats. La syntaxe utilisée dans les formules de sélection et de groupage est, par exemple, celle de SQL, pour Structured Query Language en anglais.

Le serveur de données 1 comprend des moyens de traitement de requêtes 11 pour répondre aux requêtes du service réseau de requête. Ces moyens de traitement de requêtes peuvent être installés sur un serveur rassemblant des données épidémiologiques quel que soit le format des données, sous forme de base de données ou non. Le tout est de rendre le serveur de données apte à envoyer des données sous requête d'un serveur de traitement de l'information et ce, de manière automatique. Le rattachement d'un serveur quelconque de stockage de données est alors aisé. En effet, de tels moyens de traitement de requête sont des moyens logiciels aisés à installer sur un quelconque serveur rassemblant des données épidémiologiques. Ces moyens logiciels tiennent compte du format des données stockées dans la mémoire du serveur de données et des caractéristiques des évènements épidémiologiques attendus au sein du serveur de traitement de l'information. Il est alors possible de rendre interopérables différents types de stockage et d'organisation des données épidémiologiques.

Le serveur de traitement de l'information travaille donc en temps réel en allant chercher directement les donnés épidémiologiques pour réaliser le traitement de l'information. Il ne conserve pas de données pour des traitements ultérieurs. Selon l'invention, à chaque déclenchement d'un traitement, le serveur de données est donc sollicité.

La réponse du serveur de données 1 consiste en un ensemble de données pour lesquelles on communique, pour chaque champ de cet ensemble de données, sa sémantique, c'est-à-dire le concept décrit par ce champ à partir des concepts et des relations définis dans le serveur d'ontologie et communs à la communauté de services réseaux.

La réponse du serveur de données 1 donne donc un accès aux données épidémiologiques sous la forme d'ensembles de données qui correspondent à des vues sur les évènements EE au sens de la norme ENV 13606.

La structure de ces ensembles de données respecte le modèle présenté auparavant pour les évènements EE. Cela signifie en particulier que la clé de tout ensemble de données est l'identificateur d'évènements et que tous les champs correspondant à un évènement sont présents dans l'ensemble de données.

L'utilisation des services réseaux permet au serveur de traitement de l'information 2 d'aller récupérer des données épidémiologiques relatives aux évènements EE en fonction d'un rythme de surveillance choisi au sein du service de surveillance 20, sans avoir à attendre que ces données soient exportées vers un système d'analyse par un système de déclaration de cas cliniques.

L'ensemble de la communication de données entre les serveurs se fait ainsi de manière automatique selon des caractéristiques préalablement définies pour chacun des services réseau gérant l'appel ou l'envoi de données.

Par ailleurs, comme le service de surveillance 20 ne conserve pas de photographie des données, cela évite des problèmes de cohérence des données lors des mises à jour.

Les serveurs de traitement 2 comprennent en outre un service réseau 21 pour réaliser un traitement de données épidémiologiques issues des évènements EE stockées dans la base de données relationnelle du serveur de données 1. Un tel traitement peut être numérique ou symbolique. Un seul service réseau 21 est représenté sur la figure 1 pour des raisons de clarté mais, ce sont plus généralement plusieurs services réseau de traitement de données diverses qui sont généralement mis en oeuvre.

Le service réseau de surveillance 20 héberge ainsi une bibliothèque de services réseaux et d'évènements sélectionnés mis au service de clients réseau d'interface permettant la visualisation des données. Dans cette bibliothèque, chaque service réseau est défini par une choréographie faisant appel aux services réseau de la communauté de services et chaque évènement épidémiologiques EE est décrit par l'identification du serveur de données 1 dans lequel il est stocké, un ensemble de données épidémiologiques et des informations de sélection au sein de cet ensemble de données.

L'objectif d'un serveur de traitement 2 est d'exécuter l'application des services réseaux sur les évènements choisis dans la bibliothèque et de retourner un résultat, généralement vers un client réseau vers un autre service réseau en vue d'une surveillance. Cela consiste en un chaînage d'appel à des traitements par des services réseaux. Ces traitements se font, soit sur des évènements déclarés dans la bibliothèque, soit sur des résultats d'autres traitements.

Avantageusement, le serveur de surveillance associé au service de surveillance 20 dispose de services réseaux permettant de définir de nouveaux évènements et de nouveaux services réseaux utiles pour la surveillance.

Certains de ces services réseaux sont dédiés au traitement statistique et mettent alors en oeuvre des processus statistiques parmi lesquels les études de tendance, l'identification d'aberrations spatiotemporelles, des calculs statistiques descriptifs ou interférentiels. Par exemple, des services réseau d'analyse des aberrations temporelles utilisant le EWMA (« Exponentially Weighted Moving Average » en anglais) et le CPEG (« Current Past Expérience Graph » en anglais) peuvent être mis en oeuvre. Le CPEG est avantageusement représenté graphiquement sous la forme d'une courbe reprenant les résultats du CPEG classique pour chaque point d'une fenêtre temporelle.

D'autres de ces services sont par exemple des traitements pour une analyse synthétique des répartitions des cas, suivant, par exemple, trois axes (population, temps et lieu), une visualisation des cas sur une représentation géographique, une analyse des aberrations spatio-temporelles des cas enregistrés, une visualisation temporelle des cas, l'accès à une documentation issue de la veille sanitaire.

Avantageusement, les serveurs de traitement 2 comprennent d'autres types de services réseau comme un traitement géographique à l'aide d'un système d'information géographique 23 relié à un service de gestion de requête 22. Ce système d'informations géographique 23 permet, par exemple pour un traitement de données par le service réseau du traitement statistique des données 21 de connaître les caractéristiques physiques d'un terrain localisé grâce aux données épidémiologiques. Il peut notamment s'agir de la présence d'une rivière à proximité du lieu où ont été déclarées les données épidémiologiques.

Un tel traitement géographique peut aussi permettre de présenter les données en fonction de la proximité à un lieu géographique particulier, par exemple une rivière.

Des services réseau sont avantageusement dédiés à la génération de représentations graphiques sous forme de courbes, d'histogrammes ou autres représentations graphiques.

L'ensemble des services réseaux et leurs relations sont gérées par les serveurs d'intégration 3. Pour gérer cela, les serveurs 3 comprennent notamment un annuaire des services 30 associé à des moyens d'enregistrement et de localisation des ressources en services réseau 31. Cet annuaire permet aux services réseau de se déclarer au sein de la communauté de services. Ils communiquent pour cela leur définition à l'annuaire des services 30 sur lequel ils sont alors inscrits.

Outre des données de définition selon la norme UDDI, pour « Universal Description Discovery and Integration » en anglais, l'annuaire des services est complété avec des précisions sur la couverture géographique et temporelle pour les différents serveurs de données. On note qu'il est bien sûr possible d'inscrire, dans un annuaire servant pour la définition de services réseau selon l'invention, un service réseau extérieur pour des traitements non spécifiques à l'invention.

Les serveurs 3 comprennent en outre une mémoire 33, ou serveur d'ontologie, où sont stockées des descriptions de concepts et d'objets utilisés pour la communication entre les services réseaux ainsi que des descriptions de relations entre ces concepts. Cette mémoire 33 est associée à des moyens de traitement des requêtes 34. Les concepts ainsi que les relations qui existent entre eux définissent une ontologie c'est-à-dire un langage commun partagé par la communauté des services réseau selon l'invention.

Une telle ontologie est, par exemple, formulée selon la norme OWL, pour « Web Ontology Language » en anglais, et peut s'appuyer sur la « Descriptive Ontology for Linguistic and Cognitive Engineering » en anglais (DOLCE) proposées par l'IST project 2001-33052 WonderWeb pour ses catégories supérieures.

L'ontologie décrit précisément au moins trois types de concepts : les services réseau, les évènements épidémiologiques et les attributs cliniques, biologiques ou autres associés aux évènements. Il permet un balisage sémantique des informations échangées sur le réseau 4 dont la description des services eux-mêmes.

La description des services et la choréographie sont par exemple réalisées selon la norme WSMO pour Web Service Modeling Ontology, en anglais. Chaque type de service réseau est formalisé par son traitement, les préconditions de ce traitement, ses postconditions et ses effets. Les préconditions et postconditions contiennent en particulier le format des entrées et des sorties du traitement réalisé par le service réseau.

Le serveur 3 contient aussi un concept de modèle formel pour les évènements épidémiologiques permettant de structurer l'ensemble des données épidémiologiques traitées au sein de la communauté de services réseau.

Comme vu précédemment, les événements sont associés à des attributs qui les définissent ainsi qu'à des informations sur les individus qui ont été les sujets de ces évènements.

Ces informations et attributs sont basés sur des concepts identifiés dans la norme ENV 13606 qui a été étendue afin de constituer une bibliothèque d'archétypes au sens de cette norme adaptée au domaine de l'invention.

Chacun des attributs est ainsi organisé en propriété et domaine de propriété en accord avec le langage d'ontologie utilisé, par exemple DOLCE.

Une telle organisation des attributs permet l'étiquetage sémantique de l'attribut par une propriété définie dans l'ontologie et l'étiquetage du domaine d'interprétation de cette propriété.

La communauté des services réseau est accessible à l'utilisateur via des interfaces graphiques homme-machine assurées par des clients réseau 5 dit de surveillance. Le fonctionnement des clients réseau 5 est plus précisément décrit sur la figure 2.

Le client réseau 5 comprend une interface graphique 51 que manipule un opérateur 52. Un moteur 53 du client 5 supervise les interactions et, en fonction de sa programmation et des demandes de l'utilisateur 52, organise les déclenchements des services réseau du (ou des) serveur(s) de traitement de l'information 2.

Les messages de communication avec les services réseau sont mis en forme par un générateur de messages 54. Ces messages sont ensuite adressés au serveur de traitement de l'information 2 par une interface du client 55.

Comme représenté sur le schéma fonctionnel d'un client réseau de surveillance de la figure 2, les messages 200 échangés par les services réseau entre eux et avec les clients réseau ont un contenu 201 codé en XML pour « extensible Markup Language » en anglais et enveloppé dans une enveloppe 202 suivant le protocole SOAP. A l'intérieur de l'enveloppe SOAP 202, les données épidémiologiques transitent suivant le standard européen d'échange de données XML-ENV13606 (CEN/TC251 ENV 13606) pour la structure du message, ou CEN/TS 14796 pour le typage des données.

La norme ENV 13606 permet en particulier d'identifier le demandeur et l'origine de l'échange de données, ainsi que de préciser les autorisations d'échange.

En retour, le serveur de traitement de l'information 2 renvoie un message de réponse sous un format semblable et comprenant le résultat d'un traitement de données, la réponse est ensuite décodée et mise en forme suivant une programmation prédéfinie par un module de mise en forme graphique 56, ce module 56 adresse le résultat à l'interface utilisateur 51.

La mise en forme du résultat peut être éventuellement réajustée en interaction avec l'utilisateur 52.

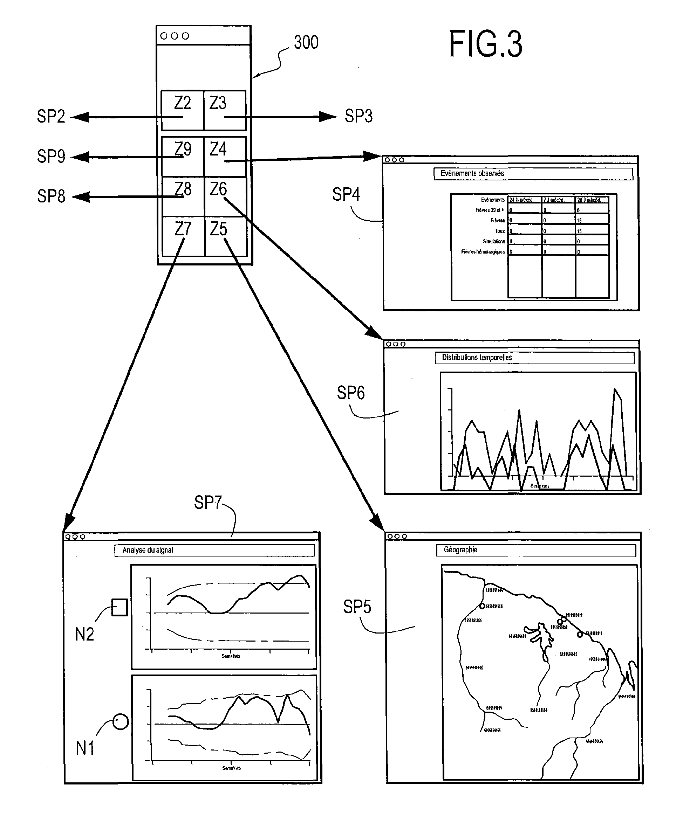

Un exemple de l'interface présentée sur les clients réseaux est décrit sur la figure 3.

L'interface proposée en exemple est basée sur le modèle des évènements épidémiologiques EE. Ce modèle permet de considérer un évènement épidémiologique comme défini sur quatre axes : la population, le temps, le lieu et le type d'évènement.

Des fenêtres graphiques affichant les données de surveillance sont paramétrées par l'utilisateur en fonction de ces axes.

Ainsi, pour l'axe temporel, l'utilisateur pourra préciser laquelle des quatre dates inclues dans le modèle des évènements épidémiologiques est choisie pour réaliser le traitement.

Une palette 300 de l'interface graphique permet d'accéder, par l'intermédiaire de plusieurs zones Z1 à Z9 de la palette 300 cliquables avec une souris à plusieurs sous-programmes SP1 à SP9 du superviseur d'interfaces 53. Dans l'exemple proposé, les sous-programmes sont donc au nombre de 9. Le client de surveillance 5 est en charge de la gestion du déclenchement des différents traitements des données épidémiologiques qui peut être périodique ou être ponctuel sous la commande d'un utilisateur.

Le rôle des clients de surveillance étant de déclencher les traitements permettant l'obtention de résultats utiles pour la surveillance épidémiologique et, le cas échéant, d'afficher ces résultats sur l'interface graphique 51, le fonctionnement ou la mise à jour d'un ou de plusieurs sous-programmes déclenche une sollicitation du serveur de traitement de l'information selon les fonctionnements présentés ci-dessus.

L'affichage d'un résultat sur l'interface graphique 51 s'accompagne avantageusement d'un rappel de l'horodatage de sa dernière mise à jour. Il s'accompagne également d'une fenêtre spatio-temporelle présentant un intervalle de temps pour la date du modèle retenue pour le traitement. Une fenêtre décrivant les attributs de l'évènement concerné peut également être affichée.

Le sous-programme SP1, non illustré sur la figure 3, permet de démarrer et d'interrompre le déclenchement itératif automatique du rafraichissement de l'affichage du client 5 par déclenchement des services réseau du serveur de traitement de l'information 2.

Lorsque ce rafraîchissement est activé, le client 5 lance un appel aux services réseau qui ont été choisis par l'utilisateur à des intervalles de temps paramétrables.

Le programme SP2, non représenté sur la figure 3, permet le paramétrage de l'interface graphique 51.

Dans l'exemple proposé, la fenêtre « évènement » permet la définition d'évènements épidémiologiques à sélectionner dans la bibliothèque du service de surveillance 20. Une telle définition se fait en donnant un identifiant du service réseau concerné donc du traitement à effectuer, un attribut d'évènement, l'ensemble de données sur lequel doit s'effectuer une sélection et des données de sélection.

Le sous-programme SP3, non représenté sur la figure 3, active un programme de contrôle de qualité des données. Il affiche le résultat de contrôles de cohérence sur les données traitées sous forme d'un ou plusieurs graphes signalant les erreurs, notamment les erreurs de déclaration, l'absence de certains attributs, les erreurs de transmission.

Le sous-programme SP4 active un affichage synthétique des données sur la base du modèle des évènements épidémiologiques. L'affichage présente les données sur deux des axes du modèle d'évènements épidémiologiques sous forme d'un tableau.

Ainsi, selon l'exemple présenté sur la figure 3 sous la forme d'une fenêtre « évènements observés », ce sous-programme SP4 permet d'afficher plusieurs tableaux où l'axe des temps est par exemple découpé sur la base des 24 dernières heures, des 7 sept derniers jours ou encore des 28 derniers jours.

Ainsi, l'affichage permet de donner les effectifs des cas déclarés en croisant sur un même tableau, les évènements et le temps, les populations et le temps, les évènements et les populations.

Le programme SP5 active une visualisation de la distribution géographique des cas, schématiquement illustrée sur la figure 3 sur le territoire de la Guyane française. Sur cet affichage, seront par exemple superposés plusieurs calques de caractères géographiques de la zone sous surveillance. Il peut s'accompagner de la possibilité de géo-localiser un point par un curseur, de positionner une coordonnée géographique, de retrouver le positionnement géographique d'une liste de noms de lieux.

Il est aussi possible de superposer des informations météorologiques issues de la requête d'un serveur Internet spécialisé par exemple sous la forme de pictogrammes et ce, pour la date en cours et, éventuellement, les jours suivants.

Des informations météorologiques relatives aux pictogrammes peuvent également être consultées en détail à partir du client réseau 5.

Il est aussi possible de superposer des photographies satellites météorologiques de la zone.

Le programme SP6, représenté par une fenêtre « distributions temporelles » sur la figure 3, active une visualisation de l'évolution temporelle des déclarations sous forme de courbe représentant un signal de surveillance. Il peut afficher simultanément l'évolution des quantités d'évènements pour plusieurs attributs d'évènements sous la forme de plusieurs courbes.

Les paramétrages des sous-programmes SP4, SP5, SP6 permettent de fixer un sous-ensemble de populations à surveiller, soit un ensemble de données à surveiller, une liste d'attributs d'évènements et une fenêtre spatio-temporelle.

Le sous-programme SP7 active une analyse des aberrations du signal de surveillance affiché dans la fenêtre du sous-programme SP6. Le sous-programme SP7 affiche un résultat pour l'ensemble des données ou pour les données correspondant à des sous-populations déterminées.

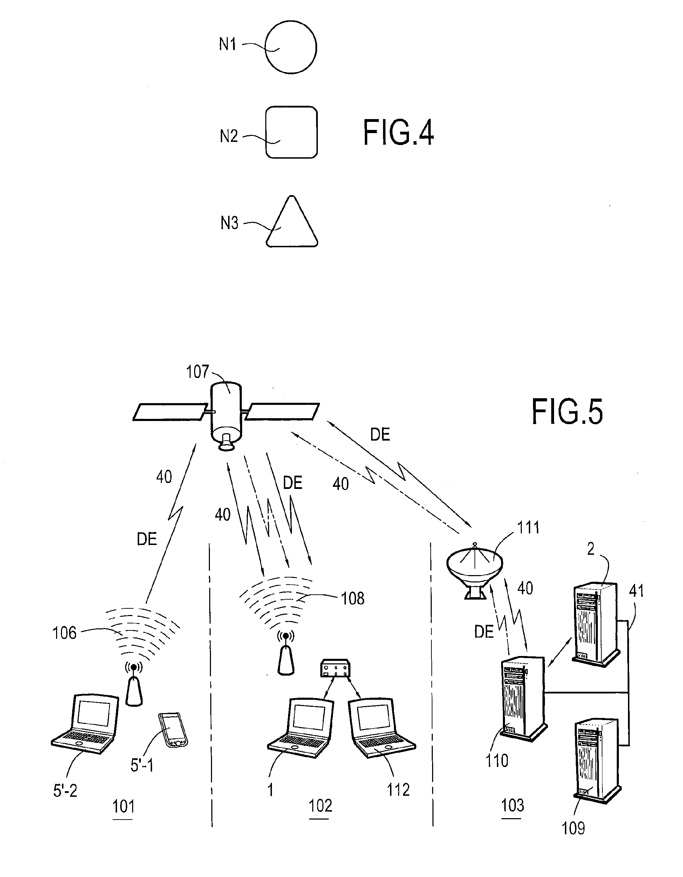

Le résultat de l'analyse de la situation courante en termes d'alerte pour les populations surveillées est résumé sous forme d'un pictogramme de niveau d'alerte dont trois exemples sont donnés sur la figure 4. Le pictogramme est avantageusement présenté à coté des courbes affichées sur la fenêtre du sous-programme SP7.

Le niveau N1 correspond à une situation normale et il est signalé, ici par un cercle. Avantageusement, un signe de risque biologique peut être placé dans le cercle et le fond du cercle peut par exemple coloré en vert. Dans ce cas, le signal reste à moins de 1,96 écarts types de la valeur attendue ou dans une zone de valeurs considérées comme normales ou usuelles.

Le niveau N2 correspond à une situation de pré-alerte symbolisée par un carré à coins arrondis. Avantageusement, de nouveau, un signe de risque biologique est dessiné dans le carré et le fond du carré est par exemple coloré en jaune orangé. Le signal est dans ce cas entre 1,96 et 3 écarts types de la valeur attendue ou dans une zone de valeurs considérées comme anormales ou inhabituelles mais pas nécessairement caractéristique d'une situation critique avérée.

Le niveau N3 correspond à une situation d'alerte avérée, symbolisée par un triangle à coins arrondis. Avantageusement, encore, un signe de risque biologique est placé dans le triangle et le fond du triangle est coloré en rouge. Dans ce cas, le signal est à trois écarts types et plus de la valeur attendue ou dans une zone de valeurs considérées comme caractéristiques d'une situation critique.

Les valeurs frontières des trois niveaux présentés définissent un modèle épidémiologique par rapport auquel est évaluée la situation dévoilée grâce aux évènements épidémiologiques.

Le sous-programme SP8, non représenté sur la figure 3, permet un affichage synthétique de la description de déclaration correspondant à un ensemble de cas. Cet affichage donne le détail des attributs des cas décomptés dans le sous-programme SP4 et est notamment appelable à partir de ce sous-programme SP4, par exemple en cliquant sur une des cases du tableau.

L'affichage du sous-programme SP8 donne avantageusement, pour chaque déclaration, la date du modèle retenu pour le traitement, sa valeur, la population d'appartenance et un résumé des informations d'intérêt. Si cela est pertinent, l'affichage peut comprendre un rappel de l'évènement participant à la définition du groupe de cas, l'effectif de ce groupe et la liste des populations concernées.

À partir de cette liste, il est possible d'accéder à une visualisation directe anonyme des données de déclarations par appel du sous-programme SP9. Ce programme SP9 permet de visualiser les données détaillées de la fiche de déclaration du cas sous une forme adéquate à la prise de décision épidémiologique.

Les valeurs attendues pour le traitement des alertes sont obtenues grâce à un historique des cas observés sur une période préalable à la mise en oeuvre du procédé selon l'invention. Les valeurs attendues sont directement saisies au sein des services réseau de traitement de données. Les valeurs attendues sont une moyenne sur une même période de l'année des cas habituellement recensés en l'absence d'épidémie.

En cas de mise en oeuvre récente de l'invention sur un nouveau théâtre d'opération, il est possible d'utiliser des valeurs attendues sur un autre théâtre d'opérations et d'affiner ces valeurs moyennes au fur et à mesure en fonction des résultats locaux. Ces valeurs attendues sont utilisées dans des traitements statistiques simples comme l'EWMA ou le CPEG.

La figure 5 montre un exemple de déploiement de l'invention dans une situation de surveillance épidémiologique d'un théâtre d'opération.

Sur cette figure, sont représentés des dispositifs utiles à l'invention et placés en des points géographiques distincts pour la surveillance épidémiologiques d'un théâtre d'opérations.

Le premier point géographique 101 est celui où se trouve la structure médicale source de données au sein du théâtre d'opérations.

Le second point géographique 102 est un centre local de traitement de données de santé situé au commandement du théâtre d'opérations.

Le troisième point géographique 103 est distant du théâtre d'opérations et est par exemple un centre d'analyse des données épidémiologiques hébergées en métropole.

Dans le contexte de la figure 5, deux configurations de client de déclaration 5' sont mises en oeuvre.

Au point géographique 101, se trouve(nt) le (ou les) système(s) de déclaration selon une première configuration adaptée à une utilisation par le personnel d'une structure médicale source de données.

Il peut s'agir par exemple d'un assistant personnel numérique 5'-1 ou encore d'un ordinateur portable 5'-2. De tels dispositifs sont adaptés aux contraintes rencontrées sur les théâtres d'opérations. L'assistant personnel numérique 5'-1 ou l'ordinateur portable 5'-2 est complété par un système de positionnement GPS intégré ou non.

Si la situation l'exige, un mode dégradé de déclaration est possible en communiquant les informations dans un message militaire transmis par voie hertzienne. Ces informations sont ensuite communiquées à un personnel médical, membre du réseau de surveillance, qui en assure la saisie sur un poste. Par ailleurs, dans les situations où le médecin d'unité ne se déplace pas, ce matériel mobile peut être remplacé par un ordinateur de bureau équipé d'un modem et/ou d'une liaison téléphonique commutée ou toute autre liaison internet.

Les systèmes de déclaration 5'-1 et 5'-2 sont connectés à un terminal satellite 106 qui assure, par l'intermédiaire d'un satellite 107 et d'un dispositif de communication satellitaire 108 situé au point géographique 102, la communication directe avec un serveur de données 1 situé au troisième point géographique 102.

L'ensemble des voies de communication satellite, notées 40 sur la figure 5, sont une partie du réseau 4.

L'assistant numérique 5'-1 ou l'ordinateur portable 5'-2 hébergent un logiciel permettant de stocker les cas transitoirement et de les transmettre automatiquement par liaison satellite protégée vers le serveur de données 1. Cette transmission est symbolisée par une flèche pleine. Le serveur de données 1 stocke alors les données épidémiologiques DE transmises.

Le dispositif de communication satellitaire 108 est par exemple relié par un réseau filaire ou optique au serveur de données 1 et le serveur de données 1 est relié par liaison satellite via le dispositif 108, le satellite 107, un dispositif de communication satellitaire 111 et un dispositif fournisseur d'accès 110 à un ou plusieurs serveurs de traitement de données 2 connecté via un réseau filaire 41, faisant partie du réseau 4. Sur ce réseau filaire 41 est avantageusement relié un serveur miroir 109 de sauvegarde par copie des données épidémiologiques. Ce serveur miroir 109 présente les mêmes caractéristiques logicielles que le serveur de données 1.

Dans la configuration de terrain représenté sur la figure 5, il se trouve que l'analyse des données épidémiologiques peut se faire soit, comme précédemment décrit, au sein des serveurs de traitement de l'information 2, par exemple placé en un point géographique distant du théâtre d'opérations 103, soit au sein du serveur de données 1 situé au commandement du théâtre d'opérations au point 102. Dans ce dernier cas, le serveur de données 1 met en oeuvre donc avantageusement des services réseaux autonomes dédiés à la surveillance, c'est-à-dire un ensemble allégé de services réseaux permettant de réaliser certains traitements statistiques localement et indépendamment des services offerts au point géographique 103.

En effet, commandé par un poste client local 112, le serveur 1 peut localement procéder à des analyses allégées. Pour cela, le serveur de données 1 communique grâce à un réseau local avec le poste client réseau 112 comprenant un client d'interface allégé adapté aux besoins du commandement santé du théâtre d'opération.

Selon l'invention, il suffit donc d'ajouter un serveur de données local 1 comprenant des moyens de traitement des requêtes envoyées par les services réseau de requête pour intégrer un nouveau théâtre d'opérations dans la surveillance épidémiologique. Il est avantageux de munir un tel serveur de données de services réseau allégés pour un traitement sommaire des données en local.

Un tel serveur de données 1 est avantageusement mobile et relié grâce à un dispositif de communication satellitaire 108 haut débit, par exemple selon la norme ISDN, pour « Integrated Services Digital Network » en anglais.

Grâce au lien satellitaire haut débit 108, le serveur 1 placé en un point 102 situé au commandement du théâtre d'opérations peut communiquer avec la communauté de services réseau présente au point géographique 103.

Dans l'exemple proposé, le client réseau 112 comprend aussi un système de déclaration adapté pour une utilisation par un centre local de traitement de données de santé. Le poste client réseau 112 héberge pour cela un logiciel de déclaration similaire à celui des médecins d'unités, intégré dans l'ordinateur portable 5'-2 ou l'assistant numérique personnel 5'-1. Il se comporte en client réseau par rapport à la communauté de services réseau selon l'invention représentée sur la figure 1. Il comprend en outre avantageusement les moyens logiciels pour réaliser une interface avec le personnel chargé de la surveillance.

Ce client allégé 112 regroupant les fonctionnalités de déclaration et de surveillance est un client autonome utilisable directement sur un réseau local situé au commandement du théâtre d'opérations connecté au serveur 110 situé au point 102. Le client 112 peut s'adresser exclusivement aux services réseau disponibles sur le serveur 1 qui, lui, peut alors faire appel à des services réseau situés, par exemple au point 103 et/ou s'adresser directement aux services réseaux présents au point géographique 103.

Dans le second cas, l'activation d'un service réseau disponible au point 103 déclenche une requête vers le serveur de données 1 pour sélection d'évènements épidémiologiques au sein du serveur de données 1 qui renvoie alors une vue sur les évènements épidémiologiques pertinents. Cet échange de requête et d'évènements épidémiologiques sont représentés par une double flèche sur la figure 5. Ensuite, avec les évènements épidémiologiques, le serveur 2 réalise une analyse des évènements et retourne un ou des résultat(s) vers le client réseau 112. La circulation des résultats d'analyse est illustrée en trait mixte sur la figure 5.

On remarque enfin que diverses mises en oeuvre peuvent être réalisées selon les principes de l'invention définis par les revendications qui suivent.

## Revendications

1. Procédé de surveillance épidémiologique mis en oeuvre à l'aide d'une communauté de services réseau déployée au sein de plusieurs serveurs [1,2,3] aptes à communiquer entre eux et dont au moins un d'entre eux, dit serveur de données [1], met en oeuvre un service réseau de stockage [10] de données épidémiologiques en provenance d'au moins un système de déclaration [5',6] de données épidémiologiques, ladite communauté pouvant être utilisée par plusieurs clients réseau [5,5'] dont au moins un client dit de surveillance [5], comprenant au moins les étapes de :
- pour le client de surveillance [5], activer un service réseau de surveillance épidémiologique [20],
- pour le service réseau de surveillance épidémiologique [20], activer au moins un service réseau apte à envoyer une requête au serveur de données [1] pour la sélection et l'envoi de données épidémiologiques,
- pour le serveur de données [1], recevoir la requête, la transférer au service réseau de stockage [10] qui réalise la sélection de données épidémiologiques, et retourner les données épidémiologiques sélectionnées,
- pour le service réseau de surveillance [20], activer un service réseau de traitement [21] apte à traiter les données épidémiologiques reçues en retour par le service réseau apte à envoyer une requête au serveur de données, et retourner le résultat du traitement au client de surveillance [5],
- pour le client de surveillance [5], afficher, le cas échéant, un résultat du traitement [SP4,SP5,SP6,SP7] sur une interface homme-machine.

2. Procédé selon la revendication 1, **caractérisé en ce que** le client de surveillance [5] active périodiquement le service réseau de surveillance [20].

3. Procédé selon l'une des revendications 1 et 2, **caractérisé en ce que** le service réseau de traitement [21] est apte à envoyer lui même une requête au serveur de données [1].

4. Procédé selon l'une des revendications 1 et 2, **caractérisé en ce que** le service réseau de surveillance [20] est apte à envoyer lui-même une requête au serveur de données [1].

5. Procédé selon l'une des revendications précédentes, **caractérisé en ce qu'**un serveur de données [1] est mis en oeuvre pour chaque site surveillé.

6. Procédé selon l'une des revendications 1 et 2, **caractérisé en ce que** les données épidémiologiques sont des n-uplets [EE] comprenant trois axes et des attributs.

7. Procédé selon l'une des revendications précédentes, **caractérisé en ce qu'**il comprend une étape préalable de saisie des données épidémiologiques en utilisant un logiciel de travail collaboratif et partagé.

8. Communauté de services réseau déployée au sein plusieurs serveurs [1,2,3] dont au moins un d'entre eux, dit serveur de données [1], met en oeuvre un service réseau de stockage [10] de données épidémiologiques en provenance d'au moins un système de déclaration de données épidémiologiques [5',6], ladite communauté pouvant être utilisée par plusieurs clients réseau [5,5'] et étant destinée à mettre en oeuvre un procédé tel que revendiqué dans les revendications 1 à 4.

9. Dispositif serveur réseau dit de traitement de l'information [2] apte à communiquer avec d'autres serveurs [1,3], l'ensemble de ces serveurs mettant en oeuvre une communauté de services réseau pouvant être utilisée par plusieurs clients réseau [5,5'], au moins un des serveurs, dit serveur de données [1], mettant en oeuvre un service réseau de stockage [10] de données épidémiologiques en provenance d'au moins un système de déclaration [5',6] de données épidémiologiques, ledit serveur de traitement de l'information [2] comprenant un service réseau de surveillance [20] apte à activer un service réseau apte à envoyer une requête au serveur de données [1] pour la sélection et l'envoi de données épidémiologiques et apte à activer au moins un service réseau de traitement [21] des données épidémiologiques sélectionnées pour traiter les données épidémiologiques.

10. Dispositif serveur réseau dit de données [1] apte à communiquer avec d'autres serveurs [2,3], l'ensemble de ces serveurs [1,2,3] mettant en oeuvre une communauté de services réseau pouvant être utilisée par plusieurs clients réseau [5,5'], le dit serveur de données [1] mettant en oeuvre un service réseau de stockage [10] de données épidémiologiques en provenance d'au moins un système de déclaration [5',6] de données épidémiologiques et des moyens de traitement [11] de requête en provenance d'un serveur de traitement de l'information [2] comprenant un service réseau de surveillance [20] apte à activer un service réseau apte à envoyer une requête au serveur de données [1] pour la sélection et l'envoi de données épidémiologiques.

11. Dispositif client réseau [5] apte à communiquer avec au moins un serveur [2] d'un ensemble de serveurs [1,2,3] mettant en oeuvre une communauté de services réseau pouvant être utilisée par plusieurs clients réseau [5,5'], le client réseau [5] comprenant des moyens pour activer un service réseau de surveillance épidémiologique apte à activer au moins un service réseau apte à envoyer une requête à un serveur de données [1], le dit serveur de données [1] mettant en oeuvre un service réseau de stockage [10] de données épidémiologiques en provenance d'au moins un système de déclaration [5',6] de données épidémiologiques, pour la sélection et l'envoi de données épidémiologiques, et apte à activer au moins un service réseau de traitement [21] apte à traiter les données épidémiologiques reçues en retour par le service réseau apte à envoyer une requête au serveur de données [1], et retourner le résultat du traitement au client de surveillance [5] et des moyens d'affichage pour afficher, le cas échéant, un résultat [SP4,SP5,SP6,SP7] du traitement des données épidémiologiques.

12. Programme d'ordinateur comportant des instructions pour l'exécution des étapes du procédé de surveillance épidémiologique selon l'une quelconque des revendications 1 à 4 lorsque ledit programme est exécuté par un ordinateur.

13. Support d'enregistrement lisible par un ordinateur sur lequel est enregistré un programme d'ordinateur comprenant des instructions pour l'exécution d'une ou de plusieurs étapes du procédé de surveillance épidémiologique selon l'une quelconque des revendications 1 à 7.
